# EUROPEAN PATENT APPLICATION

(11) **EP 2 146 354 A1**
(43) Date of publication of application: **20.01.2010**
(21) Application number: 08012701.2
(22) Date of filing: 14.07.2008
(51) Int. Cl.: G21K 1/10, G21K 5/10, G21K 5/04, A61N 5/10

(54) **Irradiation system for ion beam scanning of moving targets**

(71) Applicant: Deutsches Krebsforschungszentrum, Stiftung des öffentlichen Rechts, 69120 Heidelberg (DE)
(72) Inventor: Jäkel, Dr. Oliver, 69211 Dossenheim (DE)
(74) Representative: Reiser, Tonio Andreas

(57) **Abstract**

The present invention proposes means for fast and efficient ion beam scanning of moving targets.
Therefore, an irradiation system, comprising
- an accelerator (A),
- a beam transfer system (BT),
- a beam scanning system (BS),
- a beam monitoring system (BM) for measuring the actual position and intensity of the proton or ion beam, wherein said beam monitoring system (BM) provides a feedback to said beam scanning system (BS) and
- a patient monitoring system (PM) for monitoring the movement of the target to be treated, wherein said patient monitoring system (PM) is connected to said beam scanning system (BS) to allow an adjustment of the lateral movement of the proton or ion beam to the movement of said target;
is provided with a passive range modulator (RM) for generating a spread out Bragg Peak of the ion beam to apply a depth modulated ion beam to the target volume.

## Description

### Background of the Invention

### 1. Field of the invention

In general, the present invention relates to radiotherapy of malignant tumors. More particular, the present invention relates to an irradiation system and a method for proton or ion beam scanning of targets and especially of moving targets.

### 2. Description of the Related Art

Radiotherapy with proton and ion beams is currently investigated as highly precise and very effective treatment of malignant tumors. There are two different ways to apply the dose to the tumor:
a) the so-called passive technique which uses the interaction of the primary beam with various beam-shaping elements in order to form a treatment field which covers the target; and
b) the so-called active beam scanning, which uses two pairs of magnets to scan the primary beam over the target volume and a variation of the energy of the primary beam.

Since method a) uses hardware elements, the resulting high dose region is fixed with respect to its lateral extent and extension in depth.

Method b) relies solely on a software control. It is more flexible and it can produce nearly arbitrarily shaped dose distributions.

A special problem arises, however, when a moving organ is irradiated with an active beam-scanning system. Mainly concerned are tumors of the lung and other organs influenced by breathing motion or other internal movements. Due to interplay effects between the beam movement and the target movement it is difficult to apply a homogeneous dose to the target.

According to one approach well-known in the art, the target is only irradiated during a short time period, when the target is in a certain position, typically in the so-called deep inhale phase, when the tumor remains stable for a short time. This "Beam-gating" is described in "Minohara S. et al, Int. J. Radiat Oncol Biol Phys. 2000; 47: 1097-1103, Respiratory gated irradiation system for heavy-ion radiotherapy". The main disadvantage of this method is, that the beam is switched off most of the time and, thus, the whole irradiation procedure takes a relatively long time. This is especially true for ion beams, where mainly synchrotrons are used, which only provide a pulsed beam. Moreover, to gate the beam, a proper external signal reliably indicating, whether the internal target is in the right position, is needed.

Besides, there are approaches to modify the movement of the scanning beam in order to follow the moving organ while scanning the target volume. This technique is called "Beam-tracking" and is described in "Grözinger et al: Simulations to design an on-line motion compensation system for scanned particle beams, 2006 Phys. Med. Biol. 51 3517-3531". One problem of this technique is that the movement of the organ may change the target geometry and the radiological depth of the beam. Therefore, the optimization of the beam intensity at each beam position becomes extremely complex. Also the technical realisation is very complex. First, the beam scanning system has to compensate the movement of the target while moving the beam laterally over the target. This is relatively easy to achieve. But simultaneously, the beam energy has to be adjusted in real time for each scan position so that the penetration depth remains correct and a homogenous effect is delivered in the target. Usually, beam-tracking in longitudinal direction requires rapid variation of the beam energy. As the accelerators used in praxis only generate a monoenergetic proton or ion beam for each pulse, this is not easy to achieve. Besides, such a system is expected to be extremely safety critical.

To overcome the technical challenges of "Beam-tracking" a method called "Rescanning" has been proposed. The main idea of "Rescanning" is to repeatedly scan the target area or volume, so that statistically in the end all points in the target receive the same dose. A prerequisite for realising this method is a very fast scanning system allowing e.g. 10 scans within a second. Additionally, an extremely fast beam monitoring system is needed, which is able to detect and measure the beam position and intensity at each scan point in order to control the beam application. At least for ions, the available monitoring systems are already at the limit of sensitivity when controlling simple scanning, not to mention a rescanning. Moreover, rescanning does not lead to a conformal dose distribution.

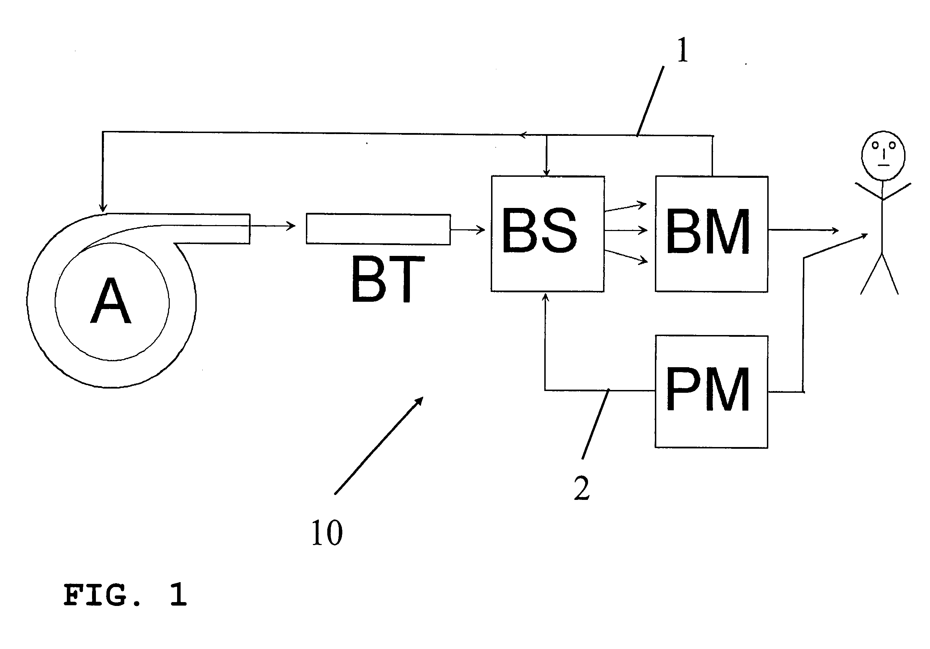

Starting point for the present invention is a prior art irradiation system 10 for proton or ion beam scanning as shown in **Fig. 1** and described herein after. In the following, the term "ion beam" is used for both "proton beam" and "ion beam", likewise.

The irradiation system 10 comprises an accelerator A for generating ion beams with an energy high enough to treat deep seated tumors.

The typical penetration depth of such an ion beam is up to 30cm. Although the energy of the accelerator A may be variable or fixed, it only produces monoenergetic ion beams per pulse with a small diameter which is typically between 1mm and 1cm. Accelerator A may be either a cyclotron or a synchrotron or a synchrocyclotron or any other type, like a laser induced particle accelerator or linear accelerator.

In case that the accelerator A can only generate a single fixed energy, like a cyclotron, a range shifter may be provided to adjust the ion range in the patient.

Irradiation system 10 further comprises a beam transfer system BT to deliver the ion beam from accelerator A to the patient. Therewith the ion beam may be transported either to a single treatment room or to several treatment rooms. The beam transfer system BT may provide only fixed horizontal, vertical or inclined beams, but may also comprise rotatable beam delivery devices, like gantries, which allow an irradiation of the patient from any direction.

For applying the ion beam to the patient, irradiation system 10 comprises a beam scanning system BS. The beam scanning system BS may dispose of two pairs of dipole magnets, which allow a magnetic deflection of the ion beam in vertical and horizontal direction to move the ion beam laterally across the target volume. Therefore an appropriate current is applied to the scanning magnets allowing the ion beam to travel with a velocity of typically 10m/s. The beam scanning system BS comprises a control computer to control the beam movement and to deliver a defined number of ions to a predefined scan spot. A beam monitoring system BM measures the actual position and intensity of the applied ion beam and gives a feedback to the beam scanning system BS in order to control the correct delivery of the ion beam and to allow the correction of the beam position and/or the delivered number of particles. To achieve a homogenous dose in the target, also the beam energy has to be corrected by an appropriate system in real time. The corresponding feedback line from the beam monitoring system BM to the beam scanning system BS and to the accelerator A is indicated by 1.

Besides, irradiation system 10 comprises a patient monitoring system PM for monitoring the movement of the target to be treated. The patient monitoring system PM is able to determine the position of the target volume with reasonable accuracy and sends this information to the control computer of the beam scanning system BS within a very short time, e.g. less than 1/100 of a second. This additional external signal is used to adapt the beam position to the target movement by adding a corresponding continuous movement to the scan movement. Thus, the ion beam is following the external signal in real-time, i.e. within less than 1/100 of a second, with variable velocity. The feedback line from the patient monitoring system PM to the beam scanning system BS is indicated as 2.

### Object of the Invention

Object of the present invention is to provide means for fast and efficient ion beam scanning of moving targets avoiding the complexity of the irradiation system described above.

### Brief Summary of the Invention

The foregoing object is achieved by an irradiation system and a method for ion beam scanning of moving targets as laid out in the independent claims. Further advantageous embodiments of the present invention are described in the subclaims and are taught in the following description.

According to the present invention a passive range modulator (RM) for depth modulating the initially monoenergetic proton or ion beam energy is added to an irradiation system, as described in connection with Fig. 1. Said passive range modulator (RM) is located between the beam transfer system (BT) and the beam scanning system (BS).

As it is state of the art, the claimed method for ion beam scanning of moving targets comprises producing at least one ion beam of a predefined depth modulation and delivering said ion beam to a target volume by a series of spots covering the maximum extent of the target volume in the direction perpendicular to the beam scanning direction. Besides, the target movement is monitored to adjust the lateral movement of the proton or ion beam to the movement of said target. However, according to the invention a spread out Bragg Peak is generated of said monoenergetic ion beam to apply a depth modulated ion beam to the target volume.

The claimed irradiation system as well as the claimed method represent an advantageous combination of the currently available beam scanning techniques with elements from the static beam delivery technique. Passive range modulators are used as depth modulators in static beam delivery systems. The present invention proposes to introduce such a modulator device into a beam scanning system and to arrange it in the beam line before the beam scanning system. Thus, the claimed irradiation system provides a depth modulated ion beam for scanning the target volume.

In beam scanning, as it is state of the art, the depth modulation is achieved by superimposing many beams with different energies. This is time consuming, as for each energy or depth layer a complete lateral scan of the beam over the target has to be performed. Using a fixed depth modulation, as proposed by the invention, only a single lateral scan is sufficient. Therefore, the irradiation is much faster. In some cases the target movement may even be neglected completely, if the scanning is fast enough. But also beam tracking becomes feasible, as the beam energy does not have to be adjusted for each scan position. Altogether, the complexity of tumor tracking in longitudinal direction is reduced significantly by the invention.

However, it should be mentioned here, that it is within the scope of the present invention to combine a relatively small depth modulation of the ion beam as claimed with a switching of the energy of the ion beam in large steps by means of the accelerator to modify the penetration depth.

As outlined above, the depth modulation of the ion beam is achieved by the passive range modulator of the claimed irradiation system. In preferred embodiments of the invention said passive range modulator comprises at least one ridge filter and/or a fast rotating modulator wheel to generate a spread out Bragg peak (SOBP) from the initially monoenergetic ion beam produced by the accelerator.

Another embodiment of the claimed irradiation system further comprises a beam shaping device for increasing the beam diameter at the patient's position. Therewith it is possible to reduce the number of scan spots and consequently, the application time in all. This beam shaping device is also located between the beam transfer system and the beam scanning system and preferably between the passive range modulator and the beam scanning system.

In fact, there are various possibilities for realisation of such a beam shaping device. However, in a preferred embodiment of the invention it comprises a scattering system composed of various material layers or a magnetic quadrupole system for defocusing the ion beam or a combination of both.

The claimed irradiation system and method are especially designed for ion beam scanning of moving targets. Therefore the irradiation system comprises a patient monitoring system, the configuration of which may vary depending on the moving organ to be monitored. A major application of such patient monitoring systems is to track a patient's respiration cycle. Therefore, the patient monitoring system may comprise a breathing belt or a laser system for detection of chest movement, and/or a spirometer to measure the air flow through the lungs. In another embodiment of the claimed irradiation system the patient monitoring system comprises a system for fluorescence imaging using x-rays to detect the position of a target directly or the position of radio-opaque markers implanted into or close to said target. In yet another embodiment the patient monitoring system comprises an electromagnetic detection system to detect the position of electromagnetic beacons implanted into or close to said target.

### Brief Description of the Several Views of the Drawings

The novel features of the invention are set forth in the appended claims. The invention itself, however, as well as a preferred mode of use, further objectives and advantages thereof, will best be understood by reference to the following detailed description of illustrative embodiments when read in conjunction with the accompanying drawings, wherein:
- **Fig. 1**: shows a diagram of an irradiation system 10 according to prior art, which has been described before in detail as starting point of the present invention;
- **Fig. 2**: shows a diagram of a first embodiment of an irradiation system 20 according to the present invention;
- **Fig. 3**: shows a dose distribution in a target volume along the beam direction (left) and transversal to the beam direction (right) achieved by irradiation system 20;
- **Fig. 4**: shows a diagram of a second embodiment of an irradiation system 40 according to the present invention; and
- **Fig. 5**: shows a dose distribution in a target volume along the beam direction (left) and transversal to the beam direction (right) achieved by irradiation system 40.

### Detailed Description of the Invention

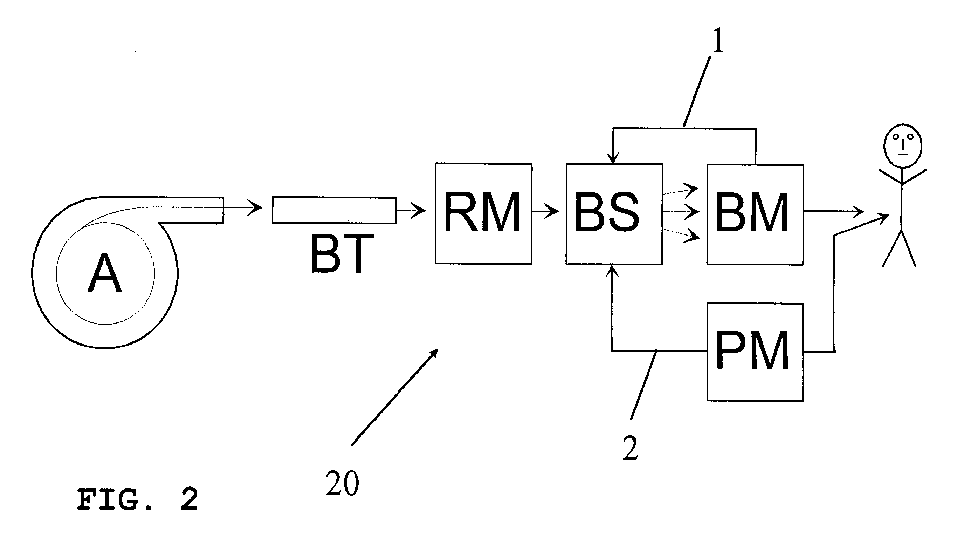

Like irradiation system 10 of Fig. 1 irradiation system 20 shown in **Fig. 2** comprises an accelerator A for producing ion beams, a beam transfer system BT for delivering the ion beam to a patient, a beam scanning system BS for moving the ion beam laterally, a beam monitoring system BM for measuring the actual position and intensity of the ion beam, and a patient monitoring system PM for monitoring the movement of the target to be treated. The beam monitoring system BM provides a feedback to the beam scanning system BS via feedback line 1 to guarantee the deliverance of a defined number of ions to a predefined scan spot, while the patient monitoring system PM is connected to the beam scanning system BS via feedback line 2 to allow an adjustment of the lateral movement of the ion beam to the movement of the target.

According to the invention irradiation system 20 further comprises a passive range modulator RM which is located between the beam transfer system BT and the beam scanning system BS. The range modulator RM allows to change the energy and thus the range of the ions in such a way, that a desired modulation of the dose is achieved along the depth through the target volume along the beam direction. The range modulation is achieved by a superposition of many beam energies with appropriate weights. The modulation occurs very fast, e.g. with less than a 1/10 of a second, or instantaneously. It may be achieved by a ridge filter or by a fast rotating modulator wheel. The dose can be modulated to be homogeneous, like it is done for proton beams, but may also be changing in order to account for variations in the relative biological effectiveness, like for ion beams.

Since irradiation system 20 is configured for ion beam scanning of moving targets the patient monitoring system PM shall be described here in more detail. The patient monitoring system PM may be configured differently depending on the type of organ that is to be treated. In order to track the respiration cycle a breathing belt can be used. The signal form the breathing belt can be correlated to the tumor position during a time resolved tomograhic imaging procedure, like 4DMRT or 4D CT prior to treatment. Another possibility is to use a laser system to detect the chest movement. Besides, a spirometer can be used to measure the air flow through the lungs and thus, determine the breathing phase. Yet another patient monitor may be a system for fluorescence imaging using X-rays during the irradiation. On the fluorescence image the position of the tumor can either be seen directly, e.g. in case of the lung, or radio-opaque markers implanted into or close to the tumor can be detected. Then, the position of the target is automatically analysed and sent to the control system of the beam scanning system. It is also possible to detect and follow the position of electromagnetic beacons using an electromagnetic detection system. The beacons can be implanted into or close to the tumor to track its position.

In the following, the use of irradiation system 20 is described, performing a method for ion beam scanning according to the present invention.

In treatment planning, the maximum penetration depth of the ion beam is determined, taking into account all possible target positions during target movement. Besides, the maximum extent of the target volume in the direction perpendicular to the ion beam direction is determined for a single position during the target movement.

In the here described example, the passive range modulator RM is built such, that the depth modulation is achieved from the minimum to the maximum extent of the target volume in the penetration depth along the ion beam direction for a fixed energy of the accelerator A. The depth modulation is fixed during the whole treatment.

The ion beam has a diameter as coming from the accelerator A. The ion beam is delivered to the target volume by series of spots covering the maximum extent of the target volume in the direction perpendicular to the ion beam direction for a single position during the movement. I.e., the scanning system will cover the target volume with one scan of the predefined scan pattern.

When the target is moving the patient monitoring system PM is sending a corresponding signal to the controller of the beam scanning system BS. This external signal is used to add a velocity to the movement of the scanning beam, so that the motion of the target volume is compensated for by the beam scanning system BS. If the speed of the system is not large enough for perfect motion compensation, the scan can be repeated several times, to allow for an averaging of irregularities in the scan process. Since the beam travels with a fast velocity and only one scan of the target volume is needed, the irradiation is very fast (about 1 s) for the whole target depending on the size of the target. Therefore, the external signal can also be used to trigger an irradiation in a breathing phase, where the target movement is minimal for a short time, typically at deep exhale. In that case no motion compensation is necessary, instead a trigger signal is sent to the scanner control.

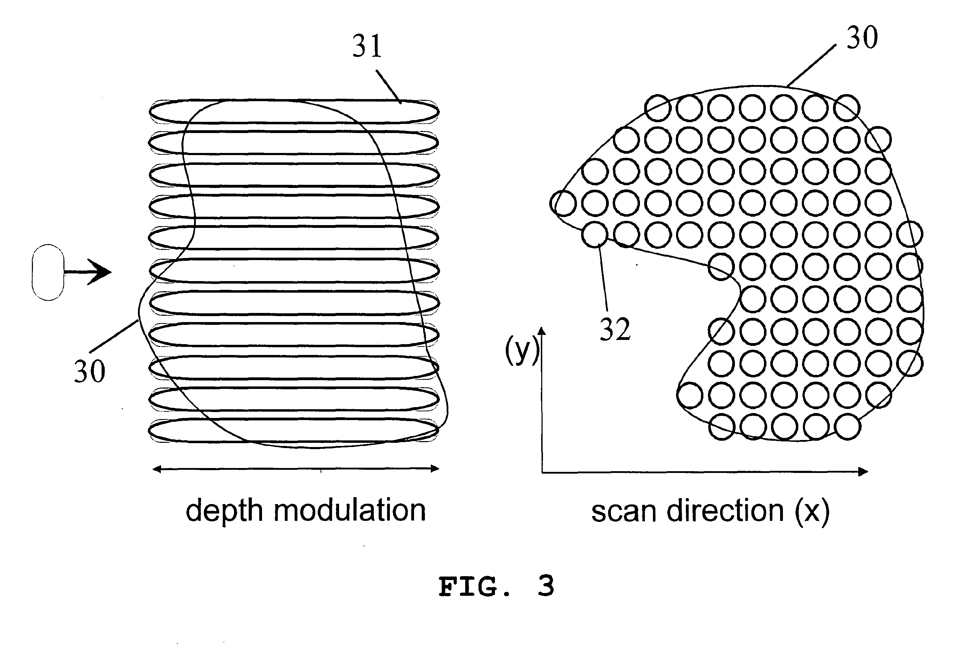

An example of the dose distribution achieved by irradiation system 20 for a static target volume 30 is shown in **Fig. 3****.** The correction for organ movement would move this dose distribution along with the target volume 30. The left part of Fig. 3 shows a section of target volume 30 along the beam direction. The ellipsoids 31 in the beam direction correspond to the fixed depth dose modulation produced by range modulator RM. In the transversal direction, which is shown in the right part of Fig. 3, the spots 32 correspond to the scan points in the x-y-plane. In the here described embodiment of the invention the dose can be adapted only in the transversal (x-y) direction of the beam.

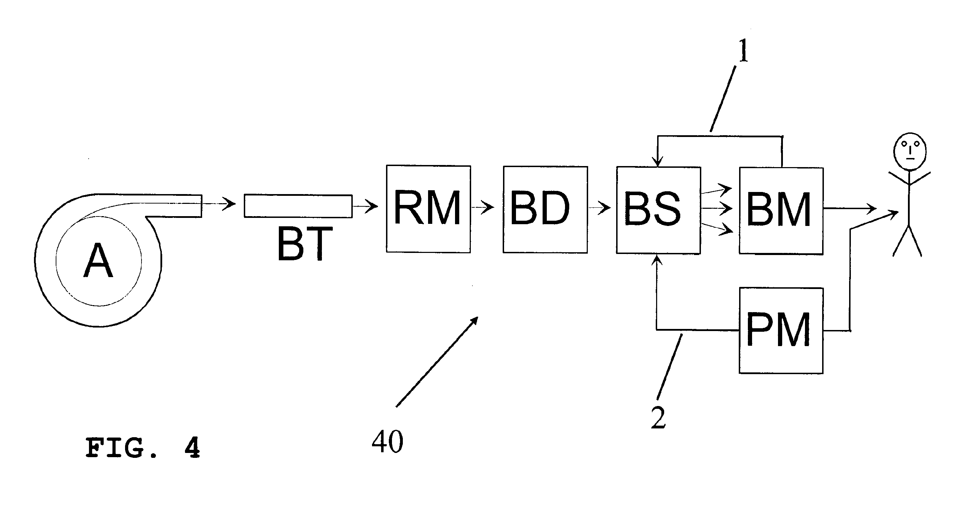

In addition to the passive range modulator RM the irradiation system 40 shown in **Fig. 4** comprises a beam shaping device BD, which is capable of increasing the diameter of the ion beam at the position of the patient. The beam shaping may be achieved by a scattering system - single or double scattering - composed of various layers of materials, or use a magnetic quadrupole system for defocusing the ion beam similar to the scanning system or a combination of both. The increased beam diameter has a typical size of 20cm - 40cm at the position of the patient.

In the here described example, the diameter of the ion beam in direction perpendicular to its direction is chosen in order to cover the maximum extent of the target volume in the patient perpendicular to the beam direction at a single fixed position of the target volume. The diameter of the beam is circular and cannot be adapted to any irregular form of the target volume. This method is therefore useful for small target volumes or volumes which are not very irregular.

In this case the target volume is not scanned by a predefined sequence of spots as described in connection with Fig. 2 and 3, but the whole target volume is covered by a single beam with the appropriate lateral extent and depth modulation. The signal from the patient monitoring system PM is used as an external signal to the controller of the beam scanning system BS in order to move the beam in accordance to the movement of the target. This form of irradiation is even faster than the method described before and can be used for very fast target motions. Also it can be used to do a fast repetition of the treatment at several positions in the cycle of movement in order to achieve at average a good coverage of the target volume.

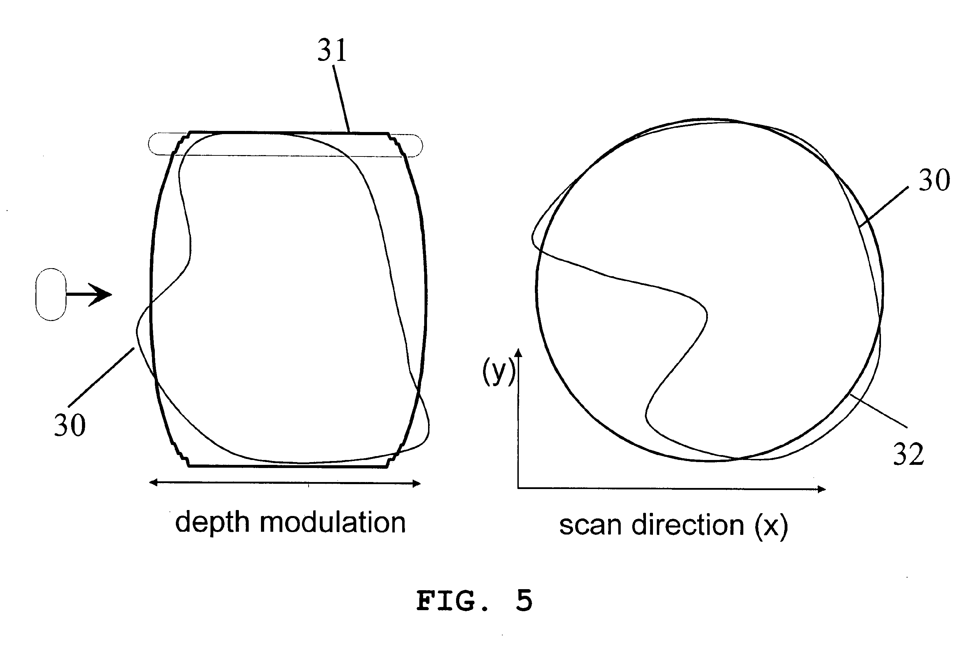

An example of the dose distribution for a static target volume 50 is shown in **Fig 5****.** The correction for organ movement would move this dose distribution along with the target volume 50. The left part of Fig. 5 shows a section of target volume 50 along the beam direction. The ellipsoid 51 in the beam direction corresponds to the fixed depth dose modulation produced by range modulator RM. In the transversal direction, which is shown in the right part of Fig. 5, the large circular beam spot 52 corresponds to the fixed broad beam in the x-y-plane. In the here described embodiment of the invention the dose can not be adapted to the target volume. But it should be mentioned here, that it is also in the scope of the present invention to perform an ion beam scanning as described in connection with Fig. 2 and 3 with an ion beam with increased diameter.

## Claims

1. Irradiation system for proton or ion beam scanning of moving targets, comprising
- an accelerator (A) for producing proton or ion beams,
- a beam transfer system (BT) for delivering the proton or ion beam to a patient,
- a beam scanning system (BS) for moving the proton or ion beam laterally,
- a beam monitoring system (BM) for measuring the actual position and intensity of the proton or ion beam, wherein said beam monitoring system (BM) provides a feedback to said beam scanning system (BS) to guarantee the deliverance of a defined number of protons or ions to a predefined scan spot, and
- a patient monitoring system (PM) for monitoring the movement of the target to be treated, wherein said patient monitoring system (PM) is connected to said beam scanning system (BS) to allow an adjustment of the lateral movement of the proton or ion beam to the movement of said target;
said irradiation system being **characterized by** a passive range modulator (RM) for depth modulating the initially monoenergetic proton or ion beam energy, wherein said passive range modulator (RM) is located between said beam transfer system (BT) and said beam scanning system (BS).

2. Irradiation system according to claim 1, wherein said passive range modulator (RM) comprises at least one ridge filter and/or a fast rotating modulator wheel to generate a spread out Bragg peak (SOBP) from an initially monoenergetic proton or ion beam.

3. Irradiation system according to claim 1 or 2, wherein said patient monitor system (PM) is used to track a patient's respiration cycle and therefore comprises
- a breathing belt, and/or
- a laser system for detection of chest movement, and/or
- a spirometer to measure the air flow through the lungs.

4. Irradiation system according to one of the claims 1 to 3, wherein said patient monitor system (PM) comprises a system for fluorescence imaging using x-rays to detect the position of a target directly or the position of radio-opaque markers implanted into or close to said target.

5. Irradiation system according to one of the claims 1 to 4, wherein said patient monitor system (PM) comprises an electromagnetic detection system to detect the position of electromagnetic beacons implanted into or close to said target.

6. Irradiation system according to one of the preceding claims, further comprising a beam shaping device (BD) for increasing the beam diameter at the patient's position, wherein said beam shaping device (BD) is located between said beam transfer system (BT) and said beam scanning system (BS).

7. Irradiation system according to claim 6, wherein said beam shaping device (BD) comprises a scattering system composed of various material layers and/or a magnetic quadrupole system for defocusing the proton or ion beam.

8. A method for ion beam scanning of moving targets,
- wherein at least one proton or ion beam of a predefined beam energy is produced and delivered to a target volume by a series of spots covering the maximum extent of the target volume in the direction perpendicular to the beam direction, and
- wherein the target movement is monitored to adjust the lateral movement of the proton or ion beam to the movement of said target;
said method being **characterized by** generating a spread out Bragg Peak of said monoenergetic ion beam to apply a depth modulated ion beam to the target volume.

9. The method according to claim 8, wherein the target volume is scanned repeatedly and wherein each single scan is carried out with a different predefined beam energy.

10. The method according to one of the claims 8 or 9, wherein the extent of the target along the beam direction as well as the target movement are taken into account for determining the maximum penetration depth along the beam direction.

11. The method according to one of the claims 8 to 10, wherein scanning is triggered in phases of minimal target movement.

12. The method according to one of the claims 8 to 11, wherein the diameter of said monoenergetic proton or ion beam is spread out perpendicular to the beam direction.

13. The method according to claim 12, wherein the extent of the target perpendicular to the beam direction are taken into account for determining the maximum beam diameter for a fixed beam energy.
